# EUROPEAN PATENT APPLICATION

(11) **EP 4 491 136 A1**
(43) Date of publication of application: **15.01.2025**
(21) Application number: 23766114.5
(22) Date of filing: 09.03.2023
(51) Int. Cl.: A61B 17/22

(54) **SHOCK WAVE ELECTRODE DEVICE AND SHOCK WAVE CATHETER SYSTEM**

(30) Priority: 09.03.2022 CN 202210234318
(71) Applicant: Jiangsu Polylive Medtech Co., Ltd., Nantong, Jiangsu 226400 (CN)
(72) Inventor: XU, Peiyao, Nantong, Jiangsu 226400 (CN); LI, Chuang, Nantong, Jiangsu 226400 (CN)
(74) Representative: Zaboliene, Reda
(86) International application number: PCT/CN2023/080629
(87) International publication number: WO 2023/169537

(57) **Abstract**

Provided in the present invention are a shock wave electrode device and a shock wave catheter system. The shock wave electrode device comprises a flexible printed circuit and an electrode assembly; the flexible printed circuit comprises a flexible substrate, a conductive pattern layer and a covering layer; the electrode assembly is integrated in the conductive pattern layer of the flexible printed circuit or on the surface of the flexible printed circuit; the electrode assembly comprises at least one pair of positive and negative electrodes, each pair of electrodes having an electrode gap between the positive electrode and the negative electrode; and the electrode assembly and the electrode gap are partially or wholly exposed on the surface of the flexible printed circuit. The shock wave catheter system provided by the present invention uses the shock wave electrode device. The shock wave catheter system manufactured by using the shock wave electrode device provided in the present invention has the advantages of miniaturization and integration, can improve the shock wave performance and stability of a catheter, and can also simplify a production and manufacturing process for a catheter, thereby improving the productivity and reducing the production cost.

## Description

### Technical Field

The present invention relates to the field of medical device technology, and in particular to a shock wave electrode device and a shock wave catheter system.

### Description of Related Art

An electrode is a component connected to both ends of a conductive medium (including solids, liquids, gases, plasmas, or vacuum) for charges to enter the medium or flow out of the medium. An electrode can be made of metal or non-metal, as long as it can exchange charges with the medium, it can become an electrode. Electrodes are divided into positive electrode and negative electrode. Positive and negative electrodes generally appear in pairs, and sometimes a pair of positive and negative electrodes connected to the same circuit are collectively referred to as electrodes. Under certain conditions, a large amount of charges accumulate at both ends of the positive and negative electrodes, generating a strong electric field. Neutral particles in the medium between the electrodes will be ionized into plasma under the action of the strong electric field, resulting in the phenomenon of discharge. The rapid temperature changes of the plasma generated during the discharge process and the surrounding medium lead to rapid volume expansion and contraction of the surrounding medium. The intense motion of particles in the medium produces pressure sound waves that propagate outward through the medium. When the speed of particle motion exceeds the propagation speed of sound waves in the medium, shock waves can be generated. Specifically, when the medium between the electrodes is liquid, the enormous energy within the plasma channel can rapidly vaporize the surrounding liquid to form bubbles, resulting in cavitation effects. The rapid expansion and rupture of cavitation bubbles accelerate the movement of particles in the medium, thereby generating powerful shock waves, a phenomenon known as the liquid electric effect.

The liquid electric effect is one of the main methods for generating shock waves, which has the advantages of high shock wave energy, high energy efficiency, fast propagation speed, and wide frequency range, and has been widely used. Liquid electric shock wave lithotripsy is a technology that generates high-intensity shock waves based on the principle of liquid electric effect for crushing hard stones, which has applications in processing, mining, and medical fields. Based on similar principles, a new type of percutaneous angioplasty has recently been used to treat calcified stenosis of blood vessels. Chinese patent CN104582597B discloses a shock wave balloon catheter with multiple shock wave sources. The shock wave balloon catheter includes a balloon that can be filled with liquid medium and an electrode assembly that can emit shock waves. This type of catheter typically places one or more sets of electrode units inside the balloon and connects to an external high-voltage circuit through one or more wires passing through the catheter. After the balloon is placed in the narrowed area of the blood vessel, pulse voltage is input into the electrodes through a wire to form liquid electricity, generating radial shock waves to break the calcification of the intima and media of the blood vessel, thereby achieving the effect of dilating the blood vessel.

### BRIEF SUMMARY OF THE INVENTION

### Technical Problem

At present, there are many electrode assemblies, complex manufacturing processes, and poor dimensional accuracy in products and technologies; the production process of electrode assemblies is complex, with low automation and inaccurate inter electrode gaps; the problems such as dense wiring of components, difficult integration and positioning, long processing time, and low product yield ultimately lead to high production cost, poor shock wave consistency, and other defects in this type of catheter. In addition, the catheter is too large and soft, with poor physical properties and vascular passability, making it still face challenges in terms of vascular adaptability, calcification removal effectiveness, and product safety, and limiting the further application of this technology in the field of vascular shaping.

### Technical Solution

In view of above, the present invention provides a shock wave electrode device, which has the characteristics of small size, high precision, simple manufacturing process, high production efficiency, easy assembly, etc., while having good shock wave performance and low production cost.

The present invention provides a shock wave electrode device. The shock wave electrode device includes:
a flexible printed circuit including a single or multiple stacked flexible substrates made of an insulating material, and a conductive pattern layer formed by printed wires and located on a surface of the flexible substrate, and/or a covering layer made of an insulating material and located on a surface of the flexible printed circuit;
an electrode assembly integrated in the conductive pattern layer inside the flexible printed circuit or integrated on the surface of the flexible printed circuit;
the electrode assembly includes at least one pair of positive and negative electrodes, each pair of positive and negative electrodes has an electrode gap formed between the positive electrode and the negative electrode; the flexible substrate and/or the covering layer forms an opening in the flexible printed circuit at a location of the electrode gap to expose the electrode assembly and the electrode gap partially or wholly from the surface of the flexible printed circuit.

Optionally, the flexible printed circuit is a soft board portion of a single-layer board, double-sided board, multi-layer board, or soft-hard combination board.

Optionally, the flexible substrate, the conductive pattern layer and the covering layer are connected to each other using a pressing process, and the flexible substrate, the conductive pattern layer and the covering layer are fixedly connected to each other through an adhesive to enhance the bonding strength.

Optionally, a surface of the covering layer is coated with an electromagnetic shielding film to increase the resistance to electromagnetic interference; a bottom surface of the flexible printed circuit is pre-integrated with a backing adhesive to improve the fixing strength.

Optionally, the insulating material of the flexible substrate and/or the covering layer includes at least one or a combination of the following:
polyimide;
polyethylene terephthalate (PET);
polyether ether ketone;
aromatic amide fiber ester;
polyvinyl chloride;
teflon.

Optionally, the electrode assembly is integrated in the conductive pattern layer of the flexible printed circuit by one or a combination of etching, deposition, rolling, and electrolysis process, or by integrating conductive components on the surface of the flexible printed circuit by one or a combination of surface mounting, soldering, and lamination process.

Optionally, according to the application scenarios and size specifications, multiple pairs of positive and negative electrodes are connected in series, in parallel, or in a mixed form through the printed wires.

Optionally, the shock wave electrode device further includes a reinforcement device; the reinforcement device is a pre-integrated structure of the flexible printed circuit or an independent structure assembled on the flexible printed circuit; the reinforcement device is located on the surface or between internal lamination layers of the flexible printed circuit; a material of the reinforcement device includes at least one or a combination of the following:
organic polymer material; and/or
inorganic non-metallic material; and/or
metal.

The present invention further provides a shock wave catheter system, including:
a catheter including a proximal catheter portion located at a proximal end of the catheter and a distal catheter portion extending axially to a distal end of the catheter; wherein the catheter includes at least one independent channel extending from the proximal catheter portion to the distal catheter portion;
an independent chamber formed by an outer space of the distal catheter portion, an expandable balloon structure, and the at least one independent channel; wherein a proximal end of the independent channel extends axially from the proximal catheter portion to be connected to the balloon structure, an interior of the independent channel and a hollow portion of the balloon structure cooperates to form an expandable independent chamber; the independent chamber is configured to fill and store a liquid medium that is isolated from human tissue and blood; the independent chamber is capable of withstanding a relative pressure of at least one standard atmospheric pressure of the liquid medium without causing leakage of the liquid medium or rupture of the independent chamber;
the shock wave electrode device described above, wherein the shock wave electrode device is located within the independent chamber; the electrode assembly is located adjacent to the distal catheter portion within the independent chamber, the flexible printed circuit of the shock wave electrode device extends towards the distal catheter portion of the catheter via the independent chamber;
a catheter joint connected to the proximal catheter portion, wherein at least one channel of the catheter joint is connected to the independent chamber for transmitting the liquid medium; the printed wires or extension wires of the shock wave electrode device form an energy transmission channel through at least one channel of the catheter joint.

Optionally, one pair or multiple pairs of positive and negative electrodes of the electrode assembly are arranged along a circumferential and/or axial direction of the catheter in the independent chamber, forming one or more electrode gaps distributed in the circumferential and/or axial direction.

Optionally, a shape of the electrode gap in the independent chamber can be one or a combination of polygonal, circular, arc-shaped, and annular shape.

Optionally, a material of the catheter and the balloon structure is one or a combination of thermoplastic polymer compounds, silicone, rubber, and polymer fiber compounds.

Optionally, the liquid medium is at least one or a combination of physiological saline, contrast agent, and/or water for injection.

Optionally, the catheter further includes an extension wire, a starting end of the extension wire is connected to the printed wire of the shock wave electrode device, the extension wire extends to an outside of the catheter via the energy transmission channel of the catheter joint, and a distal end of the extension wire is connected to an external energy generator or control handle.

Optionally, the catheter further includes a high-voltage electrical plug connected to the energy transmission channel of the catheter joint, and the flexible printed circuit or the extension wire is connected to the external energy generator or control handle via the high-voltage electrical plug to achieve energy transmission.

Optionally, the shock wave catheter system further includes:
a developing ring sleeved on the distal catheter portion or arranged in the balloon structure, configured as a developing marker for the working area of the balloon structure under X-rays.

Optionally, the developing ring is sleeved on an outer surface of the flexible printed circuit and configured for fixing the shock wave electrode device and the flexible printed circuit.

Optionally, an auxiliary support structure is further provided in the independent chamber, and the shock wave electrode device is fixed in the independent chamber by the auxiliary support structure; the auxiliary support structure is one or a combination of a support tube, stent, balloon, and probe.

Optionally, the auxiliary support structure can expand, open or move within the balloon structure to achieve the function of adjusting the position of the electrode assembly.

Optionally, the shock wave catheter system further includes:
microdevices integrated in the flexible printed circuit using printed circuits, or integrated on the surface of the flexible printed circuit using flip chip film packaging.

Optionally, the microdevice includes at least one or a combination of the following:
sensor; and/or
therapeutic device; and/or
imaging device.

### Beneficial Effects

By replacing the complex wires, electrodes, and other components and the arrangements of traditional intravascular shock wave generation devices using a flexible printed circuit, the miniaturization and integration of liquid electric generation electrodes is achieved, the manufacturing difficulty of liquid electric electrodes is reduced, the density of liquid electric electrodes is increased, and the uniformity of discharge energy release is improved. The shock wave electrode device using this type of liquid electric electrodes can accurately control the electrode gaps, accurately locate the position of the electrodes, and has the advantages of high discharge stability, balanced and controllable shock wave energy, etc. Meanwhile, components such as electrodes, wires, electrodes, and microdevices are integrated in the flexible printed circuit, avoiding problems of inaccurate positioning, insufficient insulation, and weak connections between each other, and significantly reducing the connection work between various components, simplifying the production and manufacturing process of the shock wave electrode device, and having the advantage of easy installation. The shock wave catheter system made using the shock wave electrode device provided in the present invention has the advantages of miniaturization and integration, while improving the shock wave performance and stability of the catheter, simplifying the production process of the catheter, increasing the productivity, and reducing the production cost.

### BRIEF DESCRIPTION OF THE DRAWINGS

FIG. 1 shows a schematic plan view of a shock wave electrode device provided in the present invention.
FIG. 2 shows a schematic cross-sectional view of the shock wave electrode device provided in the present invention.
FIG. 3 to FIG. 5 respectively show schematic cross-sectional views at the positions of various electrode assemblies of the shock wave electrode device provided in the present invention in a wrapping state.
FIG. 6 and FIG. 7 show schematic plan views of another shock wave electrode device provided in the present invention.
FIG. 8 shows a schematic diagram of another shock wave electrode device provided in the present invention wrapping around a cylindrical catheter.
FIG. 9 shows a schematic plan view of a shock wave catheter system provided in the present invention.
FIG. 10 shows a schematic cross-sectional view of the shock wave catheter system provided in the present invention at the position of the electrode assembly C-C.
FIG. 11 shows a schematic diagram of another connection method between a shock wave catheter system provided in the present invention and an external energy generator or a control handle.

### DETAILED DESCRIPTION OF THE EMBODIMENTS

The following specific embodiments illustrate the implementation of the present invention, and those familiar with this technology can easily understand other advantages and effects of the present invention from the content disclosed in this specification.

In the following description, reference is made to the accompanying drawings, which illustrate several embodiments of the present invention. It should be understood that other embodiments may also be used, and mechanical composition, structure, electrical, and operational changes may be made without departing from the spirit and scope of the present invention. The following detailed description should not be considered limiting, and the terms used here are only for describing specific embodiments and not intended to limit the present invention.

Although the terms "first", "second", etc. are used in this description to describe various elements in some instances, these elements should not be limited by these terms. These terms are only used to distinguish one element from another.

Further, as used in this description, the singular forms "an", "one", and "the" are intended to include the plural form as well, unless the context indicates otherwise. It should be further understood that the terms "comprise" and "include" indicate the existence of features, steps, operations, elements, components, items, categories, and/or groups, but do not exclude the existence, appearance, or addition of one or more other features, steps, operations, elements, components, items, categories, and/or groups. The terms "or" and "and/or "used here are interpreted as inclusive or imply any one or any combination. Therefore, "A, B, or C" or "A, B, and/or C" means any of the following: A; B; C; A and B; A and C; B and C; A, B and C. Exceptions to this definition only occur when a combination of components, functions, steps, or operations are inherently mutually exclusive in certain ways.

The present invention mainly focuses on a shock wave electrode device and a shock wave catheter system based on a flexible printed circuit. The relevant embodiments are usually applied in the fields of angioplasty, valvuloplasty, etc., for impacting/loosening/shattering plaques and calcified tissues of blood vessels and heart valves. However, it is expected that the device can also be used in other treatment locations and applications. In some embodiments of the present invention, the target tissue of the shock wave electrode device and the shock wave catheter system may be vascular wall plaques and calcified tissues of coronary arteries, peripheral and intracranial blood vessels. In other embodiments, the target tissue may also be plaques and calcified tissue of heart valves. In other embodiments, the target tissue is also suitable for stones or other intracavitary obstructions in the urinary, digestive, and respiratory systems.

### First embodiment

The following will take a shock wave electrode device based on a flexible printed circuit as a simple and typical example to describe and explain the structural composition, characteristic elements, and basic principles of the present invention.

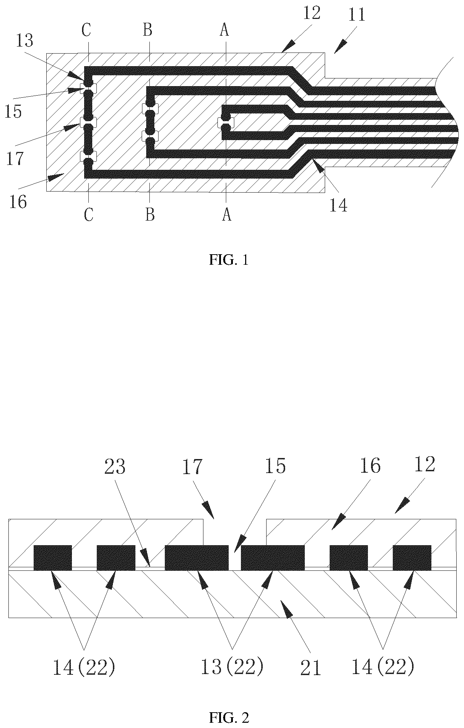

FIG. 1 shows the top view of the shock wave electrode device in this embodiment. The shock wave electrode device 11 includes a flexible printed circuit 12, and an electrode assembly 13 and printed wires 14 that are integrated in the flexible printed circuit. The electrode assembly 13 includes at least one pair of positive and negative electrodes, for example, a total of six pairs of positive and negative electrodes located at locations A-A, B-B, and C-C, with a specific electrode gap 15 existed between each pair of positive and negative electrodes. This gap is defined as the minimum value of a distance between each pair of positive and negative electrodes in the electrode assembly 13. A covering layer 16 (represented by the slash shaded area in FIG. 1) covers the upper surface of the flexible printed circuit 12 and forms an opening 17 (represented by a rounded rectangle in the figures) at the location of the electrode gap 15, so that the electrode gap 15 and part of the electrode assembly 13 are partially or wholly exposed from the surface of the flexible printed circuit 12.

FIG. 2 is a cross-sectional view of the shock wave electrode device of FIG. 1 at location A-A. As shown in FIG. 2, the flexible printed circuit 12 includes a single or multiple stacked flexible substrates 21 made of an insulating material, and a conductive pattern layer 22 (including the electrode assembly 13 and the printed wires 14), and/or a covering layer 16 made of an insulating material and covering on a surface of the flexible printed circuit, and an adhesive 23, etc. The flexible printed circuit 12 is a soft board portion of a single-layer board, double-sided board, multi-layer board, or soft-hard combination board; and a single-layer board will be taken as an example in the following for explanation and introduction of the flexible printed circuit 12. The flexible substrate 21 is a flexible insulation film made of polymer insulation materials such as polyimide, PET, and polyetheretherketone. The electrode assembly 13 and the printed wires 14 are printed on the upper surface of the flexible substrate 21 through processes such as etching, deposition, or lamination. The covering layer 16 covers the upper surface of the flexible substrate 21 that is integrated with the electrode assembly 13 and the printed wires 14. The adhesive 23 is applied between the flexible substrate 21 and the covering layer 16 to enhance the bonding strength.

When the shock wave electrode device is placed in a liquid medium, the electrode gap 15 is filled with the liquid medium due to the opening 17 of the covering layer 16. At this time, through the printed wires 14, if a high voltage current is applied between the positive and negative electrodes in the electrode assembly 13, a high-intensity electric field will be formed between the positive and negative electrodes, causing the liquid medium in the electrode gap 15 to be ionized and undergo cavitation, forming a liquid electric effect and generating shock waves.

In this embodiment, the preparation of the flexible printed circuit 12 involves printing the electrode assembly 13 and the printed wires 14 simultaneously on the surface of the flexible substrate 21 to produce the flexible printed circuit 12. In this case, the electrode assembly 13 is integrated in the conductive pattern layer 22 of the flexible printed circuit 12. In a sense, in this situation, the electrode assembly 13 can be considered as extension of the ends of the printed wires 14. However, since the opening 17 of the covering layer 16 will at least partially expose the electrode assembly 13 on the surface of the flexible printed circuit 12, and the electrode assembly 13 is in the form of a typical structure with an electrode pair and the electrode gap 15, it can be clearly distinguished whether any segment of the printed circuit is an electrode assembly 13 or a printed wire 14 through the above methods.

In some other embodiments, the flexible printed circuit 12 can be prepared by printing pin contacts of the electrode assembly 13 and other optional microdevices, and the printed wires 14 on the surface of the flexible substrate 21. After the initial pattern of the flexible printed circuit 12 is made, the electrode assembly 13 and other optional microdevices are integrated on the surface of the initial pattern using processes such as SMT, soldering, or lamination, and finally the flexible printed circuit 12 with the electrode assembly 13 is prepared. In this case, the electrode assembly 13 is usually located on the surface of the flexible printed circuit 12.

The above two methods or their variants and combinations can be used as general methods for preparing the shock wave electrode device 11. However, different application scenarios and limitations should be considered in order to select a corresponding preparation method. For example, in scenarios where there is a high demand for electrode miniaturization, the method in this embodiment should be preferred, while in scenarios where high shock wave energy and longer electrode lifespan are required, the second method should be preferred.

Generally, the width of the electrode gap 15 is usually 0.025mm to 2.5mm, such as 0.05mm, 0.1mm, 0.12mm, 0.15mm, 0.18mm. In this embodiment, the nominal width of the electrode gap 15 is 0.2mm.

As an example, the surface of the covering layer 16 of the flexible printed circuit 12 can be coated with an electromagnetic shielding film, and the bottom surface of the flexible printed circuit 12 can be pre-integrated with a backing adhesive.

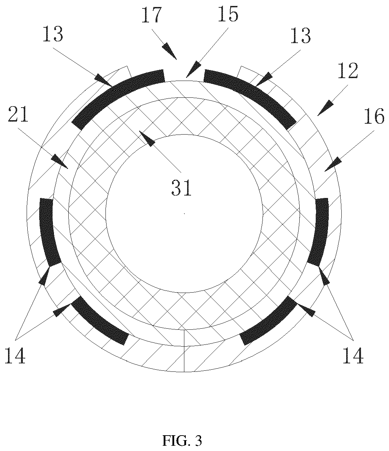

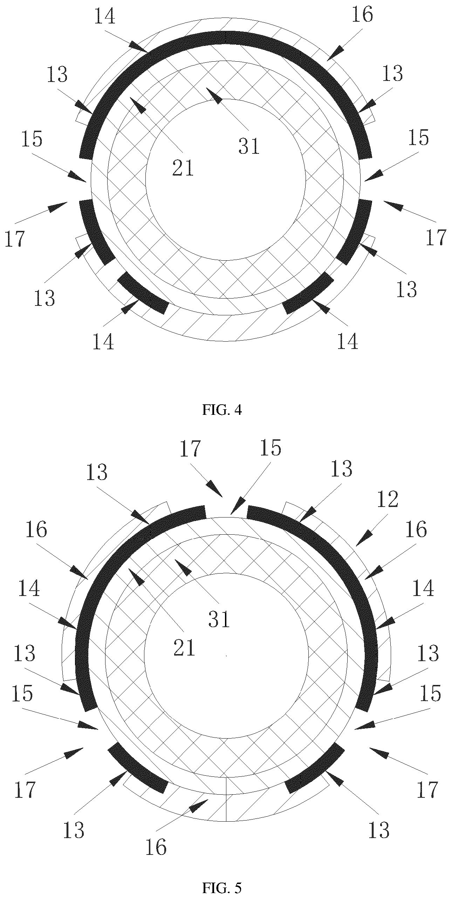

FIG. 3 to FIG. 5 respectively show the circumferential cross-sectional views of the shock wave electrode device in this embodiment at the electrode locations A-A, B-B, and C-C, as a supplement to FIGS. 1 and 2. This situation can be understood as an example of the shock wave electrode device being attached and fixed to an auxiliary support structure. In this embodiment, the auxiliary support structure is a cylindrical support tube 31 (equivalent to a catheter as described below). For clarity, the adhesive 23 in FIG. 2 is not shown in FIG. 3, which also conforms to the situation where the adhesive 23 may not be applicable in some other embodiments, and the flexible substrate 21 is directly laminated with the covering layer 16.

It should be specifically noted that the flexible printed circuit 12 can also be designed in any closed shape made of line segments, arcs, etc. The shape of the flexible printed circuit 12 is suitable for carrying the electrode assembly 13, the printed wires 14 and the covering layer 16. The specific shape of the flexible printed circuit 12 should correspond to the design of the shock wave electrode device 11, and will not be repeated here. Moreover, due to the basic characteristics of flexibility and bendability of the flexible printed circuit 12, the shock wave electrode device 11 in the present invention can be attached to the surface of an auxiliary support structure of any shape and imitate the shape of the structure. The description of the external shape of the auxiliary support structure in this embodiment and other embodiments of the present invention cannot constitute a limitation on the structural appearance of the shock wave electrode device 11 and the auxiliary support structure in the present invention.

### Second embodiment

The structural composition, characteristic elements, and basic principles of the shock wave electrode device in the present invention are explained based on the above embodiment. This embodiment provides a shock wave electrode device based on a flexible printed circuit with greater engineering value. Compared with the first embodiment mentioned above, the flexible printed circuit 12 in this embodiment has the same material composition and the controlled width of the electrode gap 15. Therefore, only the structural characteristics of a typical specification in this embodiment will be described in detail.

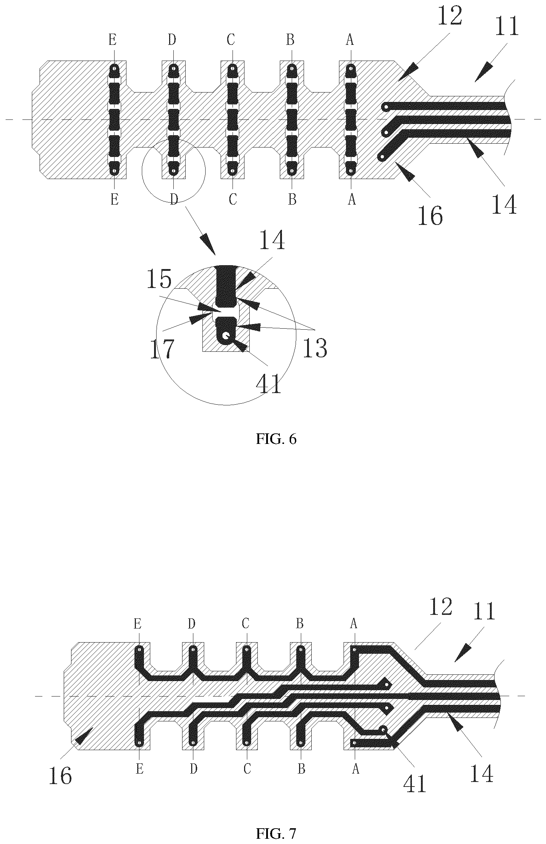

The flexible printed circuit 12 of the shock wave electrode device 11 in this embodiment is constituted by a single-layer flexible substrate 21 and two conductive pattern layers 22 located on the upper and lower surfaces of the flexible substrate 21. The electrode assembly 13 and some printed wires 14 are integrated in the conductive pattern layer 22 on the upper surface of the flexible substrate 21, and the conductive pattern layer 22 on the lower surface of the flexible substrate 21 is formed by printed wires 14 for achieving circuit conduction. The covering layer 16 covers the surface of the flexible substrate 21 integrated with the electrode assembly 13 and the printed wires 14, and forms an opening 17 at the electrode gap 15, to expose the electrode gap 15 and a portion of the electrode assembly 13 from the surface of the flexible printed circuit 12. The unfolded front top view of the shock wave electrode device 11 with the typical specification in this embodiment is shown in FIG. 6.

As an example, the electrode assembly 13 of the shock wave electrode device 11 is comprised of five groups of positive and negative electrodes connected in parallel, which are located at locations A-A, B-B, C-C, D-D, and E-E of the shock wave electrode device 11. Each group of positive and negative electrodes is comprised of four pairs of positive and negative electrodes, which are evenly spaced from each other and connected in series, to form four electrode gaps 15 which are evenly spaced from each other, as shown in FIG. 6. The direction along A-B-C-D-E is defined as the length direction of the shock wave electrode device 11, and when the shock wave electrode device 11 is symmetrically wrapped along the length direction and connected end-to-end from A-A to E-E, the four electrode gaps 15 can be evenly distributed at 90° angle in the circumferential direction.

The unfolded back view (bottom view) of the shock wave electrode device 11 is shown in FIG. 7. The conductive pattern layer 22 on the back side is formed by the printed wires 14, which mainly connect the electrodes to achieve conductivity of the electrode circuit. The hollow circular holes that appear at the same locations in both FIG. 6 and FIG. 7 are through holes 41, which are used to connect the printed wires 14 of the upper and lower conductive pattern layers 22 to form a conductive circuit.

As an example, in this embodiment, the shock wave electrode device 11 has a total of twenty pairs of positive and negative electrodes, and corresponding twenty electrode gaps 15. Due to the openings formed in the covering layer 16 corresponding to the electrode gaps 15, the shock wave electrode device 11 has a total of twenty shock wave generation points under the liquid electric effect.

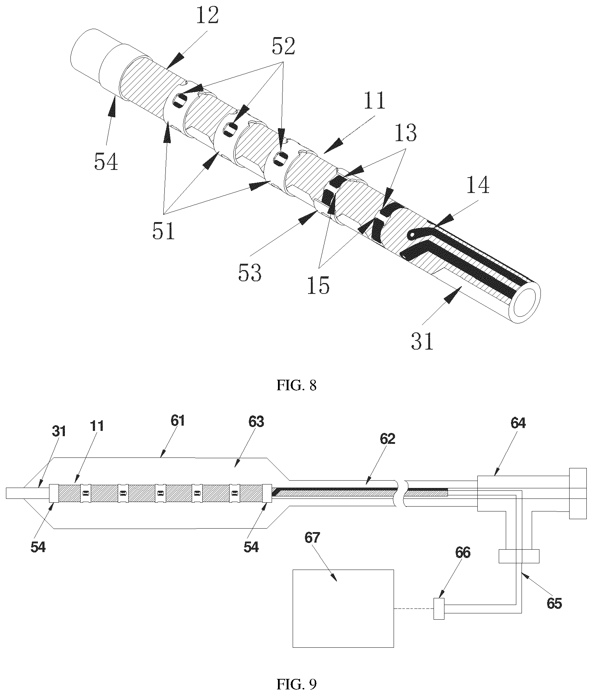

When the shock wave electrode device in this embodiment is applied to a shock wave catheter system, it needs to be wrapped around a cylindrical catheter 31, as shown in FIG. 8. In the typical specification of this embodiment, the shock wave electrode device 11 can be in a wrapping state along the length direction such that A-A to E-E are connected end-to-end, thereby completely covering the surface of the cylindrical catheter 31, with the four electrode gaps 15 at each location evenly distributed in the circumferential direction of the catheter 31. Generally, in this embodiment, for the shock wave electrode devices 11 with other specifications, when there are two pairs of electrodes in the same cross-section, the two electrode gaps 15 are distributed and spaced at 180° interval in the circumferential direction of the catheter 31; when there are three electrode gaps 15 in the same cross-section, the electrode gaps 15 are distributed and spaced at 120° interval in the circumferential direction of the catheter 31, and so forth. The above interval setting method can maintain force balance of the catheter 31 during electrode discharge.

As an example, this embodiment has a reinforcement device with an independent structure, and the reinforcement device is a metal circular ring 51. The inner diameter of the circular ring 51 is slightly greater than the diameter of the shock wave electrode device in the wrapping state of the shock wave electrode device 11, so that the circular ring 51 can be sleeved and fixed on the surface of the shock wave electrode device. The circular ring 51 is evenly distributed with four openings 52 in the circumferential direction, such that the electrode gaps 15 of the shock wave electrode device are aligned with the openings 52 of the circular ring 51 in the sleeving state to effectively expose the electrode gaps 15. This design is used to reduce the mechanical impact of shock waves generated by the electrode assembly 12 on the flexible printed circuit 11, improve the reliability of the shock wave electrode device 11 and shock wave catheter system, and increase their service life. FIG. 8 shows the situation where part of the electrode assembly 13 and electrode gaps 15 are sleeved by the circular rings 51, wherein the circular ring 53 is a circular ring 51 in the quarter section for better illustrating the positional relationship between the electrode assembly 13, the electrode gaps 15, and the circular ring 51.

As an example, in this embodiment, after the shock wave electrode device 11 is sleeved on the catheter 31, the distal end of the flexible printed circuit 12 is fixed using a developing ring 54.

For other specifications of this embodiment, the size of the diameter of the catheter 31 and the composition of the electrode assembly 13, as well as the number and arrangement of the electrode gaps 15, can be determined according to the actual situations, wherein the electrode gap 15 can be one or a combination of polygonal, circular, arc-shaped, and annular shape, and will not be further described in this embodiment.

### Third embodiment

Based on the shock wave electrode device in the second embodiment, this embodiment provides an implementation of a shock wave catheter system with the shock wave electrode device 11 described above, and a detailed explanation of this implementation is as follows.

The shock wave catheter system in this embodiment is shown in FIG. 9. The main components of the system include a shock wave electrode device 11 described above, a catheter 31, a developing ring 54, a balloon 61 and its independent channel 62, an independent chamber 63, a catheter joint 64, an extension wire 65, and a high-voltage electrical plug 66. The catheter 31 includes a proximal catheter portion located at the proximal end of the catheter 31 and a distal catheter portion extending axially to the distal end of the catheter 31. The balloon 61 in the form of an expandable balloon structure is arranged on the outer layer of the distal catheter portion of the catheter 31. A hollow portion of the balloon 61 and the independent channel 62 cooperate to form an expandable independent chamber 63. The flexible printed circuit 11 is disposed inside the independent chamber 63. When injecting a liquid medium into the independent chamber 63, the balloon 61 can withstand a relative pressure of at least one standard atmospheric pressure of the liquid medium without causing leakage of the liquid medium or rupture of the balloon 61.

As an example, the shock wave electrode device 11 is installed in the independent chamber 63 located at the distal catheter portion of the catheter 31, and the flexible printed circuit 12 extends towards the proximal catheter portion of the catheter 31 via the independent channel 62. The preferred solution, as in the second embodiment, is to wrap and integrate the shock wave electrode device 11 at the distal catheter portion of the catheter 31 along the length direction, such that the shock wave electrode device 11 completely covers the surface of the cylindrical catheter 31, with the electrode gaps 15 at each location evenly distributed in the circumferential direction of the catheter 31. The installation methods of the shock wave electrode device 11 can include adhesion, soldering, riveting, binding, buckle, etc. In this embodiment, the shock wave electrode device 11 is fixedly installed on the catheter 31 by adhesion.

As an example, in this embodiment, the shock wave electrode device 11 is provided with four electrode gaps 15 evenly distributed in the circumferential direction at the same cross-section of the catheter 31. Considering its generality, in order to maintain force balance during discharge of the shock wave electrode device 11, multiple electrode gaps 15 on the same cross-section of the catheter 31 should be arranged at equal angular intervals from each other in the circumferential direction of the catheter 31. For the shock wave catheter system made using the shock wave electrode device 11 of different specifications in the second embodiment, when there are two pairs of electrodes in the same cross-section, the two electrode gaps 15 are distributed and spaced at 180° interval in the circumferential direction of the catheter 31; when there are three electrode gaps 15 in the same cross-section, the electrode gaps 15 are distributed and spaced at 120° interval in the circumferential direction of the catheter 31, and so forth. The above interval setting method can maintain force balance of the catheter 31 during electrode discharge.

As an example, the hollow portion inside the balloon 61 extends axially from the distal end of the catheter 31 to the proximal end of the catheter 31 through the independent channel 62, and is connected to the catheter joint 64. The liquid medium can be injected into the independent chamber 63 formed between the balloon 61 and the catheter 31 through an injection chamber in the catheter joint 64. The liquid medium includes water, physiological saline, contrast agent, or a mixed solution of the above liquids. The extension wire 65 passes through the catheter joint 64 to connect the shock wave electrode device 11 with an external energy generator 67.

As an example, the developing ring 54 is installed on the catheter 31, and the developing ring 54 is sleeved on the catheter 31 and located inside the balloon 61. The sleeved developing ring 54 is located on both ends of the flexible printed circuit 12 to reinforce and protect the flexible printed circuit 12. When the above-mentioned shock wave catheter system is fed into the lesion site by a guide wire, the position of the shock wave electrode device 11 can be confirmed through the developing ring 54.

Optionally, the shock wave catheter system further comprises an auxiliary support structure for installing the shock wave electrode device 11, the auxiliary support structure is provided in the independent chamber 63, and the shock wave electrode device 11 is installed on the auxiliary support structure. The auxiliary support structure is usually one or a combination of a support tube, stent, balloon, and probe.

Optionally, the support structure can expand, open, or move within the balloon structure to achieve the function of adjusting the position of the electrode assembly 13.

Optionally, the shock wave electrode device further includes microdevices, which are integrated in the flexible printed circuit using printed circuits, or integrated on the surface of the flexible printed circuit using flip chip film packaging. The number of the microdevices can be any natural number such as 0, 1, 2, 3, etc. The microdevices can be sensors (such as temperature sensors, potential sensors, pressure sensors, impedance sensors, etc.), therapeutic devices (such as thermocouples, ablation devices, etc.), imaging devices (such as intravascular ultrasound imaging transducers, etc.), or a combination thereof.

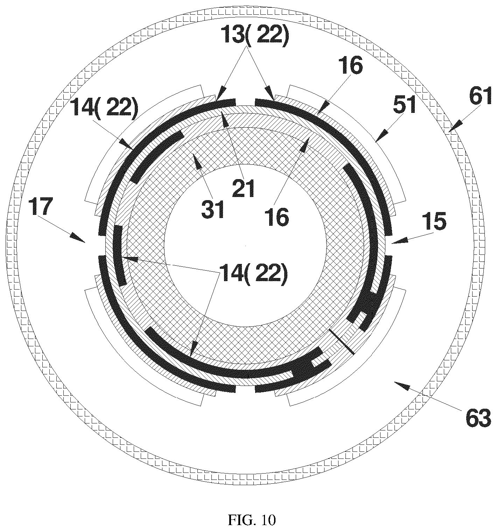

The shock wave electrode device 11 in this embodiment is the same as the shock wave electrode device in the second embodiment of the typical specifications. Referring to FIG. 6 and FIG. 7, the flexible printed circuit 12 of the shock wave electrode device 11 is constituted by a single-layer flexible substrate 21 and two conductive pattern layers 22 located on the upper and lower surfaces of the flexible substrate 21. The electrode assembly 13 and some printed wires 14 are integrated in the conductive pattern layer 22 on the upper surface of the flexible substrate 21, and the conductive pattern layer 22 on the lower surface of the flexible substrate 21 is formed by printed wires 14 for achieving circuit conduction. The electrode assembly 13 and the printed wires 14 are printed on the surface of the flexible substrate 21 through processes such as etching, deposition, or lamination. The covering layer 16 covers the surface of the flexible substrate 21 integrated with the electrode assembly 13 and the printed wires 14, and forms an opening 17 at the electrode gap 15, to expose the electrode gap 15 and a portion of the electrode assembly 13 from the surface of the flexible printed circuit 12. The shock wave electrode device 11 is fixed to the catheter 31 by adhesion, and the upper surface of the flexible printed circuit 12 should be exposed to the internal space of the independent chamber 63, such that the electrode gap 15 can be in contact with the medium in the independent chamber 63. FIG. 10 shows a schematic cross-sectional view of the shock wave catheter system at the location of the electrode assembly 13.

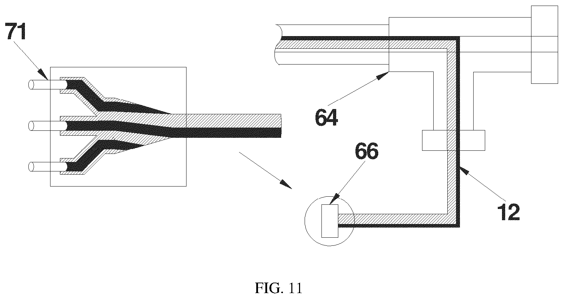

As an example, the shock wave catheter system includes an extension wire 65. A starting end of the extension wire 65 is connected to the distal end of the printed wire 14 of the shock wave electrode device 11 and extends to the outside of the catheter 31 via an energy transmission channel of the catheter joint 64. The other end of the extension wire 65 is connected to an external energy generator or control handle 67 via a high-voltage electrical plug 66. In some embodiments, a longer flexible printed circuit 12 can be directly extended to the high-voltage electrical plug 66 through the catheter joint 64, and directly connected to channel pins 71 in the high-voltage electrical plug 66, for ultimately being connected to the external energy generator or control handle 67. The partial schematic diagram of this connection method is shown in FIG. 11.

Generally, the electrode gaps 15 can be separately connected to an energy generator 67 to achieve individual control of each shock wave generation point, for applying different voltages or currents according to the severity of the lesion site. Optionally, multiple electrode gaps 15 can be connected together in series/parallel/mixed form and then connected to the energy generator 67 to jointly generate shock waves, for achieving a wider range of therapeutic effects.

The above embodiments are only illustrative of the principles and effects of the present invention, and are not intended to limit the present invention. Any changes of equivalent structures or equivalent processes made using the content of the specification and drawings of the present invention, or directly or indirectly applied in other related technical fields, are also included in the scope of patent protection of the present invention.

### Industrial applicability

The shock wave electrode device in the present invention replaces the complex wires, electrodes, and other components and the arrangements of traditional intravascular shock wave generation devices through a flexible printed circuit, achieving the miniaturization and integration of liquid electric generation electrodes, reducing the manufacturing difficulty of liquid electric electrodes, increasing the density of liquid electric electrodes, and improving the uniformity of discharge energy release. The shock wave electrode device using this type of liquid electric electrodes can accurately control the electrode gaps, accurately locate the position of the electrodes, and has the advantages of high discharge stability, balanced and controllable shock wave energy, etc. Meanwhile, components such as electrodes, wires, electrodes, and microdevices are integrated in the flexible printed circuit, avoiding problems of inaccurate positioning, insufficient insulation, and weak connections between each other, and significantly reducing the connection work between various components, simplifying the production and manufacturing process of the shock wave electrode device, and having the advantage of easy installation. The shock wave catheter system made using the shock wave electrode device provided in the present invention has the advantages of miniaturization and integration, while improving the shock wave performance and stability of the catheter, simplifying the production process of the catheter, increasing the productivity, and reducing the production cost.

## Claims

1. A shock wave electrode device, wherein the shock wave electrode device comprises:
a flexible printed circuit comprising a single or multiple stacked flexible substrates made of an insulating material, and a conductive pattern layer formed by printed wires and located on a surface of the flexible substrate, and/or a covering layer made of an insulating material and covering on a surface of the flexible printed circuit;
an electrode assembly integrated in the conductive pattern layer inside the flexible printed circuit or integrated on the surface of the flexible printed circuit;
the electrode assembly comprises at least one pair of positive and negative electrodes, each pair of positive and negative electrodes has an electrode gap formed between the positive electrode and the negative electrode; the flexible substrate and/or the covering layer forms an opening in the flexible printed circuit at a location of the electrode gap to expose the electrode assembly and the electrode gap partially or wholly from the surface of the flexible printed circuit.

2. The shock wave electrode device as claimed in claim **1,** wherein the flexible printed circuit is a soft board portion of a single-layer board, double-sided board, multi-layer board, or soft-hard combination board.

3. The shock wave electrode device as claimed in claim **1,** wherein the flexible substrate, the conductive pattern layer and the covering layer are connected to each other using a pressing process, and the flexible substrate, the conductive pattern layer and the covering layer are fixedly connected to each other through an adhesive.

4. The shock wave electrode device as claimed in claim **1,** wherein a surface of the covering layer is coated with an electromagnetic shielding film; a bottom surface of the flexible printed circuit is pre-integrated with a backing adhesive.

5. The shock wave electrode device as claimed in any one of claims **1** to **4,** wherein the insulating material of the flexible substrate and/or the covering layer comprises at least one or a combination of the following:
polyimide;
polyethylene terephthalate;
polyether ether ketone;
aromatic amide fiber ester;
polyvinyl chloride;
teflon.

6. The shock wave electrode device as claimed in claim **1,** wherein the electrode assembly is integrated in the conductive pattern layer of the flexible printed circuit by one or a combination of etching, deposition, rolling, and electrolysis process, or by integrating conductive components on the surface of the flexible printed circuit by one or a combination of surface mounting, soldering, or lamination process; multiple pairs of positive and negative electrodes are connected in series, in parallel, or in a mixed form through the printed wires.

7. The shock wave electrode device as claimed in any one of claims **1** to **4,** wherein the shock wave electrode device further comprises a reinforcement device; the reinforcement device is a pre-integrated structure of the flexible printed circuit or an independent structure assembled on the flexible printed circuit; the reinforcement device is located on the surface or between internal lamination layers of the flexible printed circuit; a material of the reinforcement device comprises at least one or a combination of the following:
organic polymer material; and/or
inorganic non-metallic material; and/or
metal.

8. A shock wave catheter system comprising:
a catheter comprising a proximal catheter portion located at a proximal end of the catheter and a distal catheter portion extending axially to a distal end of the catheter; wherein the catheter comprises at least one independent channel extending from the proximal catheter portion to the distal catheter portion;
an independent chamber formed by an outer space of the distal catheter portion, an expandable balloon structure, and the at least one independent channel; wherein a proximal end of the independent channel extends axially from the proximal catheter portion to be connected to the balloon structure, an interior of the independent channel and a hollow portion of the balloon structure cooperates to form an expandable independent chamber; the independent chamber is configured to fill and store a liquid medium that is isolated from human tissue and blood; the independent chamber is capable of withstanding a relative pressure of at least one standard atmospheric pressure of the liquid medium without causing leakage of the liquid medium or rupture of the independent chamber;
the shock wave electrode device as claimed in any one of claims **1** to **7,** wherein the shock wave electrode device is located within the independent chamber; the electrode assembly is located adjacent to the distal catheter portion within the independent chamber, the flexible printed circuit of the shock wave electrode device extends towards the proximal catheter portion of the catheter via the independent channel;
a catheter joint connected to the proximal catheter portion, wherein at least one channel of the catheter joint is connected to the independent chamber for transmitting the liquid medium; the printed wires of the shock wave electrode device form an energy transmission channel through at least one channel of the catheter joint.

9. The shock wave catheter system as claimed in claim **8,** wherein one pair or multiple pairs of positive and negative electrodes of the electrode assembly are arranged along a circumferential and/or axial direction of the catheter in the independent chamber, forming one or more electrode gaps distributed in the circumferential and/or axial direction; a shape of the electrode gap in the independent chamber can be one or a combination of polygonal, circular, arc-shaped, and annular shape.

10. The shock wave catheter system as claimed in claim **8,** wherein a material of the catheter and the balloon structure is one or a combination of thermoplastic polymer compounds, silicone, rubber, and polymer fiber compounds; the liquid medium is at least one or a combination of physiological saline, contrast agent, and/or water for injection.

11. The shock wave catheter system as claimed in any one of claims **8** to **10,** wherein the catheter further comprises an extension wire, a starting end of the extension wire is connected to the printed wire of the shock wave electrode device, the extension wire extends to an outside of the catheter via the energy transmission channel of the catheter joint, and a distal end of the extension wire is connected to an external energy generator or control handle.

12. The shock wave catheter system as claimed in claim **11,** wherein the catheter further comprises a high-voltage electrical plug connected to the energy transmission channel of the catheter joint, and the flexible printed circuit or the extension wire is connected to the external energy generator or control handle via the high-voltage electrical plug to achieve energy transmission.

13. The shock wave catheter system as claimed in claim **12,** wherein the shock wave catheter system further comprises:
a developing ring sleeved on the distal catheter portion or arranged in the balloon structure, configured as a developing marker for the working area of the balloon structure under X-rays; the developing ring is sleeved on an outer surface of the flexible printed circuit and configured for fixing the shock wave assembly device and the flexible printed circuit.

14. The shock wave catheter system as claimed in claim **13,** wherein an auxiliary support structure is further provided in the independent chamber, and the shock wave electrode device is fixed in the independent chamber by the auxiliary support structure; the auxiliary support structure is one or a combination of a support tube, stent, balloon, and probe; the auxiliary support structure can expand, open or move within the balloon structure to achieve the function of adjusting the position of the electrode assembly.

15. The shock wave catheter system as claimed in claim **14,** wherein the shock wave catheter system further comprises:
microdevices integrated in the flexible printed circuit using printed circuits, or integrated on the surface of the flexible printed circuit using flip chip film packaging;
the microdevice comprises at least one or a combination of the following:
sensor; and/or
therapeutic device; and/or
imaging device.
